(19) Europäisches Patentamt
European Patent Office
Office européen des brevets

(11) Veröffentlichungsnummer: **0 112 571**
**B1**

(12) # EUROPÄISCHE PATENTSCHRIFT

(45) Veröffentlichungstag der Patentschrift: **05.08.87**

(51) Int. Cl.⁴: **C 12 N 9/80**, C 12 Q 1/34

(21) Anmeldenummer: **83113075.2**

(22) Anmeldetag: **23.12.83**

(54) **Verfahren und Reagenz zur Bestimmung von N-Carbamoylsarcosin und hierfür geeignetes neues Enzym.**

(30) Priorität: **27.12.82 DE 3248145**

(43) Veröffentlichungstag der Anmeldung: **04.07.84 Patentblatt 84/27**

(45) Bekanntmachung des Hinweises auf die Patenterteilung: **05.08.87 Patentblatt 87/32**

(84) Benannte Vertragsstaaten: **AT BE CH DE FR GB IT LI LU NL SE**

(56) Entgegenhaltungen:
**EP - A - 0 066 552**

**ENZYME HANDBOOK Bd II (1969) Springer Verlag Berlin, S. 650-651.**

(73) Patentinhaber: **Boehringer Mannheim GmbH, Sandhofer Strasse 116, D-6800 Mannheim 31 (DE)**

(72) Erfinder: **Deeg, Rolf, Dr., Hirtenstrasse 7, D-8139 Bernried (DE)**
Erfinder: **Röder, Albert, Dr., Tiefentalweg 8, D-8124 Seeshaupt (DE)**
Erfinder: **Siedel, Joachim, Dr., Bahnhofstrasse 6, D-8131 Bernried (DE)**
Erfinder: **Gauhl, Helmgard, Mitterfeld 4, D-8132 Tutzing (DE)**
Erfinder: **Ziegenhorn, Joachim, Dr., Ina-Seidel-Weg 1, D-8130 Starnberg (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

EP 0 112 571 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren und ein Reagenz zur Bestimmung von N-Carbamoylsarcosin unter Verwendung des neuen Enzyms N-Carbamoylsarcosin-Amidohydrolase sowie die Gewinnung des letzteren.

Es wurde gefunden, dass Mikroorganismen die Fähigkeit besitzen, Creatinin in N-Carbamoylsarcosin umzuwandeln. Für die Erforschung dieses Abbauweges wäre ein spezifisches Verfahren zur Bestimmung von N-Carbamoylsarcosin erwünscht. Ausserdem könnte ein derartiges Verfahren auch für die Bestimmung des Creatinins selbst eingesetzt werden durch vorangehende Umwandlung in N-Carbamoylsarcosin.

Der Erfindung liegt daher die Aufgabe zugrunde, eine spezifische Bestimmung des N-Carbamoylsarcosins zu ermöglichen.

Gelöst wird diese Aufgabe durch ein Verfahren zur Bestimmung von N-Carbamoylsarcosin-Amidohydrolase, welche sich aus N-Carbamoylsarcosin, spezifisch Sarcosin gemäss, nachstehender Formel bildet:

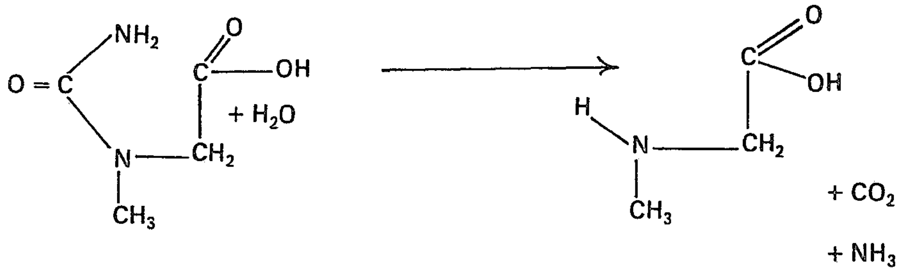

erhältlich aus Mikroorganismen wie Arthrobacter DSM 2563, DSM 2564, Micrococcus spec. DSM 2565 oder Moraxella DSM 2562, die auf N-Methylhydantoin gezüchtet werden.

Die Erfindung beruht auf der Auffindung des neuen Enzyms N-Carbamoylsarcosin-Amidohydrolase, abgekürzt CSH, welches spezifisch Sarcosin bildet unter Abspaltung von $CO_2$ und $NH_3$ gemäss nachstehender Formel:

N-Carbamoyl-Sarcosin | N-Carbamoyl-sarcosin-Amidohydrolase | Sarcosin

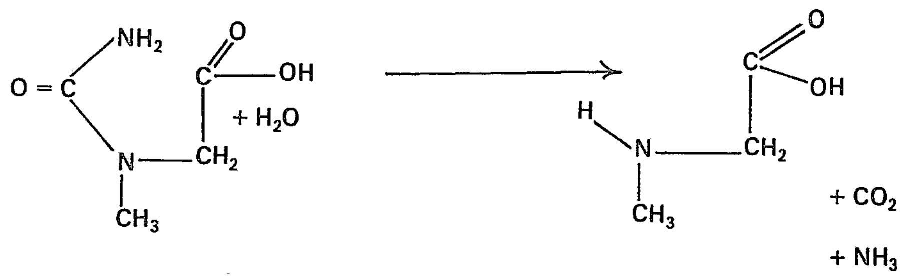

Beim erfindungsgemässen Verfahren gebildetes Sarcosin kann nach bekannten Methoden bestimmt werden, beispielsweise unter Verwendung von Sarcosin-Dehydrogenase und Messung von gebildetem NADH oder mittels Sarcosinoxidase und Messung von gebildetem $H_2O_2$ oder verbrauchtem $O_2$. Bevorzugt wird die Bestimmung unter Verwendung von Sarcosinoxidase und Messung von gebildetem $H_2O_2$. Letzteres kann sowohl titrimetrisch als auch potentiometrisch, polarographisch und colorimetrisch sowie enzymatisch bestimmt werden. Bevorzugt werden hierunter die enzymatischen Methoden unter Verwendung von Katalase oder Peroxidase (POD), da diese äusserst spezifisch und zuverlässig sind. Die Bestimmung mittels Katalase erfolgt in Gegenwart von β-Detonen wie z.B. Acetylaceton und Methanol bzw. Äthanol oder Methylenglycol. Die Bestimmung mit Peroxidase erfolgt in Gegenwart eines oder mehrerer Chromogene. Beispiele für geeignete Chromogene sind 2,2′-Aminobenzthiazolinsulfonsäure (ABTS), das Indikatorsystem nach Trinder (Ann. Clin. Biochem. 6 [1969], 24–27), bei dem Phenol oder ein anderer aromatischer Alkohol bzw. ein aromatisches Amin mit 4-Aminophenazon (4-Aminoantipyrin) oder einem 4-Aminophenazonderivat oxidativ zu einem Farbstoff gekuppelt werden. Geeignete aromatische Alkohole bzw. Amine sind beispielsweise p-Chlorphenol, Aminophenole, Naphthol und seine Derivate, Naphthylamin und seine Derivate, Aminochinoline, Hydroxychinoline, Dihydroxyphenylessigsäure und ähnliche. Anstelle von 4-Aminophenazon können ≙ 4-Aminophenazon-Derivat, Phenylendiaminsulfonsäure, Methylbenzothiazolonhydrazon (MBTH), sulfoniertes Methylbenzothiazolonhydrazon (S-MBTH) und deren Derivate verwendet werden.

Die erfindungsgemässe Bestimmung des N-Carbamoyl Sarcosins erfolgt zweckmässig in gepufferter Lösung. Geeignete pH-Werte liegen zwischen etwa 6 und 9.

Ein weiterer Gegenstand der Erfindung ist ein Reagenz zur Bestimmung von N-Carbamoylsarcosin, welches dadurch gekennzeichnet ist, dass es N-Carbamoylsarcosin-Amidohydrolase und ein System zum Nachweis von Sarcosin enthält.

Ein bevorzugtes Reagenz der oben bezeichneten Art enthält als System zum Nachweis von Sarcosin Sarcosinoxidase, Peroxidase und Chromophor.

Ein weiteres bevorzugtes System zum Nachweis von Sarcosin besteht aus Sarcosindehydrogenase und NAD.

Ausserdem enthalten die erfindungsgemässen Reagenzien noch Puffer, pH 6 und 9 und gegebenenfalls ein oberflächenaktives Mittel.

Ein weiterer Gegenstand der Erfindung ist das neue Enzym N-Carbamoylsarcosin-Amidohydrolase (CSH).

Folgende Eigenschaften wurden für dieses Enzym bestimmt:

Stabilität

Stabilitätsoptimum: pH 6 bis 6,5; gute Stabilitätseigenschaften liegen in Phosphatpuffer vor, beste Stabilität wird bei Zusatz von Glycerin erzielt.

Erhitzung CSH 20 mg/ml in 50% Glycerin pH 6,5 für 20 Minuten:

| | |
|---|---|
| Restaktivität %: | 40°C/100 |
| | 50°C/ 48 |
| | 60°C/ 24 |
| | 80°C/ 0 |

Bei +4°C in 50% Glycerin ist der Aktivitätserhalt nach vier Wochen 100%.

Spezifität

Creatinin, Creatin, Sarcosin, N-Methylhydantoin und Hydantoin werden nicht umgesetzt.

Michaelis Konstante für N-Carbamoylsarcosin bie 25°C, pH 8,0, 0,1 M Trispuffer 3,3 bis $3,8 \times 10^{-3}$M.

In 0,1 M $K_4P_2O_7$ Puffer pH 8,5: 5,0 bis $7,1 \times 10^{-3}$M.

pH-Optimum

Das pH-Optimum hängt von der Puffersubstanz ab. Bei $K_4P_2O_7$ Puffer wurde es zu 8,5, bei Trispuffer und Hepespuffer zu 8,0 bestimmt.

Molekulargewicht

MG = ca. 40 000. Bestimmung durch Disc.-Elektrophorese.

Inhibitoren

5 µg Merthiolat/ml hemmt zu 95%.

Gleichgewicht

Das Gleichgewicht wurde mit 0,5 U/ml in 90 mM/l Tris pH 8,0 bestimmt:

$$\frac{\text{N-Carbamoylsarcosin}}{\text{Sarcosin}} = \frac{12,5\text{ mM}}{<0,001\text{ mM}} = >1 \times 10^4$$

Die Gewinnung von CSH erfolgt durch Züchtung geeigneter Mikroorganismen und Gewinnung des Enzyms aus der Biomasse oder der Kulturbrühe. Als besonders geeignet erwiesen sich Mikroorganismen der Gattungen Arthrobacter und Moraxella. Die besten Ergebnisse wurden erzielt mit Arthrobacter DSM 2563, DSM 2564, Micrococcus spec. DSM 2565 oder Moraxelle DSM 2562.

Die Mikroorganismen können in Form einer Zellsuspension für das erfindungsgemässe Bestimmungsverfahren eingesetzt werden. Vorzugsweise verwendet man jedoch für die Erfindung ein gereinigtes Enzympräparat. Zu dessen Herstellung schliesst man einen auf N-Methylhydantoin gezüchteten Mikroorganismus mit einem die Aufarbeitung lohnenden Gehalt an N-Carbamoylsarcosin-Amidohydrolase auf und trennt vom Unlöslichen ab. Für den Aufschluss eignen sich die gebräuchlichen Methoden wie Hochdruckdispersion, Ultraschall, Desintegrationsmühle oder Lysozym-Zusatz. Dem erhaltenen klaren Extrakt werden 0,3 bis 0,5 Gew.% Polyäthylenimin zugesetzt, der gebildete Niederschlag abgetrennt und der Überstand mit Wasser verdünnt, wobei das Enzym ausfällt und abgetrennt werden kann.

Zur Feinreinigung kann das so gewonnene Enzym den üblichen Methoden der Enzymfeinreinigung unterworfen werden. Vorzugsweise wird das wie oben beschrieben hergestellte Enzympräparat in geeignetem Puffer gelöst und zwischen 1,3 und 2,2 M-Ammoniumsulfat fraktioniert. Durch Chromatographie über Phenylsepharose® und DE-52-Cellulose sowie Gelfiltration über Sephacryl® S 200 erhält man ein von Fremdaktivitäten freies Präparat mit einer spezifischen Aktivität von 2 U/mg Protein, in einer Ausbeute von etwa 70%, bezogen auf die im Zellextrakt vorgefundene Menge. Das so gewonnene Enzym kann ohne Aktivitätsverlust in 50%iger Glycerin/Wasser-Lösung, pH 6,5, bei +4°C mindestens ein halbes Jahr aufbewahrt werden.

Die Erfindung ermöglicht es, N-Carbamoylsarcosin spezifisch zu bestimmen. Eine solche Methode ist ein wichtiger Beitrag insbesondere für die Aufklärung des Creatinin-Stoffwechsels. In Verbindung mit der Überführung von Creatinin in N-Carbamoylsarcosin lässt sich das erfindungsgemässe Verfahren auch direkt für die Creatininbestimmung einsetzen.

Die folgenden Beispiel erläutern die Erfindung weiter:

Beispiel 1

Bestimmung von N-Carbamoylsarcosin

Prinzip:

N-Carbamoylsarcosin + $H_2O \xrightarrow{CSH}$ Sarcosin + $CO_2$ + $NH_3$

Sarcosin + $O_2$ + $H_2O \xrightarrow{Sarc\text{-}OD}$ Glycin + Formaldehyd + $H_2O_2$

$H_2O_2$ + 4-Aminophenazon $\xrightarrow{Peroxidase}$ Farbstoff + 2 $H_2O$ + Phenol-Derivat

Reagenz:

0,1 mol/l Trispuffer, pH 8 }
0,2 mmol/l 4-Aminophenazon } Farbbildungssystem
2,0 mmol/l 2,4,6-Tribrom-3-Hydroxy-benzoat }
0,4% Triton X-100 (oberflächenaktives Mittel als Löslichkeitsverbesserer)
2,0 U/ml Carbamoylsarcosin-Amidohydrolase
2,0 U/ml Sarcosinoxidase
2,0 U/ml POD

Probe:

wässrige Lösung mit 0–35,5 µmol/l N-Carbamoylsarcosin

Testansatz:

2 ml Reagenz
100 µl Probe

Testdurchführung:

Probe und Reagenz mischen, 30 Minuten bei Raumtemperatur stehenlassen, in Küvette überführen und Extinktion der Probe gegen Reagenzleerwert bei = 546 nm ablesen.

Ergebnis:

Es ergibt sich eine lineare Abhängigkeit der Extinktion von der N-Carbamoylsarcosin-Konzentration in der Probe.

Beispiel 2

A) 10 ml eines N-Methyl-Hydantoin enthaltenden Mediums (Zusammensetzung s.u.) werden mit Arthrobacter DSM 2563, BMTU 2194-1, von Schraegagar gleicher Zusammensetzung beimpft und im 100 ml Erlenmeyerkolben ca. 24 Stunden bei 28°C und 160 Rpm geschüttelt. Anschliessend wird zu 1% in Medium gleicher Zusammensetzung weitergeimpft, so dass 500 ml dieser Kultivierungsstufe für einen 50-l-Fermenter vorliegen (5 × 1 ml der 1. Kultur in je 100 ml/500 ml Erlenmeyer, 24 Stunden/28°C und 160 Rpm).

Diese 500 ml werden in 50 l Medium gleicher Zusammensetzung überführt und in einem Fermenter, der mit 2 Blattrührern/Schikanen ausgerüstet ist, mit 100 l Luft/Stunde und 500 Rpm bei 28°C weiterfermentiert.

Nach 10 bis 12 Stunden beginnt die Bildung der N-Methyl-Hydantoin-abbauenden Enzyme, die bis zum Ende der logarithmischen Wachstumsphase anhält. Nach 18 Kultivierungsstunden findet die Ernte statt. Die Mikroorganismen werden in einer Kühlzentrifuge abgetrennt, die Biomasse anschliessend in 0,1 M Phosphatpuffer pH 8 gewaschen, erneut zentrifugiert und eingefroren. Es wurden auf diese Weise aus 50 l Kultur ca. 800 g Biomasse gewonnen.

Mediumzusammensetzung:

Pro 1: 7 g $Na_2NPO_4 \times 2\,H_2O$, 3 g $KH_2PO_4$, 0,5 g $MgSO_4 \times 7\,H_2O$, 10 g N-Methyl-Hydantoin (steril filtriert), 5 g Hefe-Extrakt/Difco, 1 ml Spurenlösung 1*, 0,1 ml Spurenlösung 2**, 1 ml Vitaminlösung*** (steril filtriert).

*Spurenlösung 1:

100 mg $MnCl_2 \times 4\,H_2O$, 100 mg $FeCl_3 \times 6\,H_2O$, 100 mg $CaCl_2 \times 2\,H_2O$ in 100 ml bidest. Wasser gelöst und sterilisiert. 1 ml dieser Lösung auf 1 l Medium.

**Spurenlösung 2:

1 mg $CuCl_2 \times 2\,H_2O$, 1 mg $ZnCl_2$, 1 mg $(NH_4)_2MoO_4$, 1 mg $CoCl_2 \times 6\,H_2O$ in 1000 ml Wasser gelöst und sterilisiert. 0,1 ml dieser Lösung auf 1 l Medium.

*** Vitaminlösung:

0,1 mg Biotin, 0,1 mg Pyridoxol, 0,1 mg Pyridoxamin × HCl, 0,1 mg PABS, 1,0 mg Riboflavin, 1,0 mg Nicotinamid, 1,0 mg Folsäure, 10,0 mg Thiamin × HCl in 100 ml bidest. Wasser gelöst und steril filtriert. 1 ml dieser Lösung auf 1 l Medium.

B) Die gewaschenen Zellen (1 kg Trockengewicht) werden in 50 mM/l Kaliumphosphat-Puffer, pH 6,5, auf 15 l aufgenommen und bei ca. 600 bar (atü) in der Hochdruckdispersion aufgeschlossen. Der Zellrückstand wird abgetrennt. Der klare Extrakt enthält die CSH. Er wird mit 4% 10%iger Polyäthylenimin (G-35-)Lösung, pH 6,5, versetzt. Nach Abtrennung der Nucleinsäuren wird das Enzym durch Verdünnung mit Wasser gefällt. Der geringe Proteinniederschlag wird mit 50 mM/l Kaliumphosphat-Puffer, pH 6,5, aufgenommen und zwischen 1,3 und 2,2 M/l mit Ammoniumsulfat fraktioniert. Durch anschliessende hydrophobe Chromatographie an Phenylsepharose® wird Sarcosinoxidase vollständig entfernt.

Im abnehmenden Gradienten 20 mM/l Kaliumphosphat-Puffer, pH 6,5, enthaltend 1,0 M/l Ammoniumsulfat, bis 20 mM/l Kaliumphospaht-Puffer, pH 6,5, wird CSH bei ca. 0,4 M/l Ammoniumsulfat eluiert.

Das Eluat wird gegen 20 mM/l Kaliumphosphat-Puffer, pH 6,5, kalt dialysiert und auf eine mit gleichem Puffer equilibrierte Säule mit DE-52-Cellulose aufgetragen. Die CSH wird durch

einen ansteigenden Gradienten mit obigem Puffer zwischen 50 bis 500 mM/l NaCl eluiert. Das Eluat wird durch Ultrafiltration konzentriert. Nach der anschliessenden Gelfiltration in 0,1 M Kaliumphosphat-Puffer, pH 6,5, (Sephacryl-S-200®) erhält man die CSH in 53% Ausbeute und einer spezifischen Aktivität von 2,1 U/mg. Das Enzym kann ohne Verlust in 50% Glycerin-Lösung pH 6,5, bei +4°C über 6 Monate aufbewahrt werden.

Einzelheiten zeigt nachfolgende Tabelle.

Trennung und Reinigung von CSH aus 1 kg Trockenmasse Arthrobacter

| Schritt | CSH | | | |
|---|---|---|---|---|
| | Protein in g | KU | U/mg | Ausbeute % |
| Zellaufschluss (Extrakt) | 200 | 28 | 0,14 | 100 |
| Zellfragmente = entleerte Zelle | - | 0,5 | - | 1,7 |
| Zellextrakt + G-35, 2. Niederschlag | 54 | 27 | 0,50 | 96 |
| 1,3 - 2,2 M AS/ Niederschlag | 40 | 24 | 0,60 | 86 |
| Phenylsepharose®-Eluat | 13,8 | 22 | 1,60 | 79 |
| DE-52-Cellulose-Eluat | 10 | 18 | 1,80 | 64 |
| Endpräparat nach Gelfiltration | 7,1 | 15 | 2,10 | 53 |

Beispiel 3

Aus einem Kulturansatz Moraxella DSM 2562 (BMTU 2193-1), gezüchtet auf N-Methylhydantoin, wie in Beispiel 2 A) beschrieben, werden 180 g Trockenmasse gewonnen. Die CSH wird ähnlich wie im Beispiel 1 B) aufgereinigt. Jedoch werden die Zellen statt in der Hochdruckdispersion mit 0,05% Lysozym (g/g Trockenmasse) bei pH 7,0 lysiert.

Isolierung von CSH aus 180 g Trockenmasse Moraxella

| Schritt | CSH | | | |
|---|---|---|---|---|
| | Protein in g | KU | U/mg | Ausbeute % |
| Zell-Lyse-Extrakt | 28,4 | 3,5 | 0,12 | 100 |
| Zellfragmente | - | - | - | - |
| Zellextrakt + G-35, 2. Niederschlag | 7,7 | 3,4 | 0,44 | 97 |
| 1,25 - 2,1 M AS/ Niederschlag | 5,7 | 3,3 | 0,58 | 94 |
| Phenylsepharose®-Eluat | 2,0 | 3,3 | 1,65 | 94 |
| DE-52-Cellulose-Eluat | 1,2 | 2,5 | 2,1 | 71 |
| Endpräparat nach Gelfiltration | 1,2 | 2,4 | 2,0 | 68 |

Beispiel 4

Bestimmung von CSH mit o-Dianisidin

Prinzip:

$$\text{N-Carbamoylsarcosin} + H_2O \xrightarrow{\text{CSH}} \text{Sarcosin} + CO_2 + NH_3$$

$$\text{Sarcosin} + O_2 + H_2O \xrightarrow{\text{Sarc-OD}} \text{Glycin} + \text{Formaldehyd} + H_2O_2$$

$$H_2O_2 + \text{o-Dianisidin} \xrightarrow{\text{POD}} \text{Farbstoff} + 2\,H_2O$$

Lösungen:

1. Tris-Puffer 0,1 mol/1; pH 8,0
2. ortho-Dianisidin-Lösung; 66 mg o-Dianisidin-hydro-chlorid in 10 ml Puffer (1) lösen.
3. Testpuffer: 1,0 ml Lösung 2 in 99 ml Lösung 1 pipettieren und mischen. Eine Woche bei +4°C haltbar.
4. Peroxidase: 2 mg POD (Boehringer Mannheim GmbH 108090, Reinheitsgrad I) in 1 ml $H_2O$ lösen.
5. Sarcosinoxidase: 100 U/ml gereinigte Sarc-OD (Uricase- und Katalase-frei) in $H_2O$ lösen.
6. N-Carbamoylsarcosin 0,25 mol/l

Probe:
Verdünnungen mit kalten 50 mmol/l $KPO_4$-Puffer ph 6,5.

Ausführung:
436 nm; 25°C; V = 2,00 ml; d = 1 cm; ε = 8,3 $cm^{2x\mu mol-1}$

In Küvette pipettieren

| | | |
|---|---|---|
| Puffer | (3) | 1,80 ml |
| POD | (4) | 0,01 ml |
| Sarc-OD | (5) | 0,05 ml |
| Carbamoylsarcosin | (6) | 0,10 ml |

mischen, ca. 10 Minuten warten, dann mit Probe starten

| | |
|---|---|
| Probe | 0,04 ml |

mischen, 10 Minuten laufen lassen und ΔE/min aus der linearen Phase berechnen

$$\text{Berechnung: } \frac{\Delta E/\text{min} \times 2 \times \text{Verdünnung}}{8{,}3 \times \text{eingesetzte ml Probe}} = \text{U/ml}$$

**Patentansprüche**

1. N-Carbamoylsarcosin-Amidohydrolase, welche aus N-Carbamoylsarcosin spezifisch Sarcosin gemäss nachstehender Formel bildet:

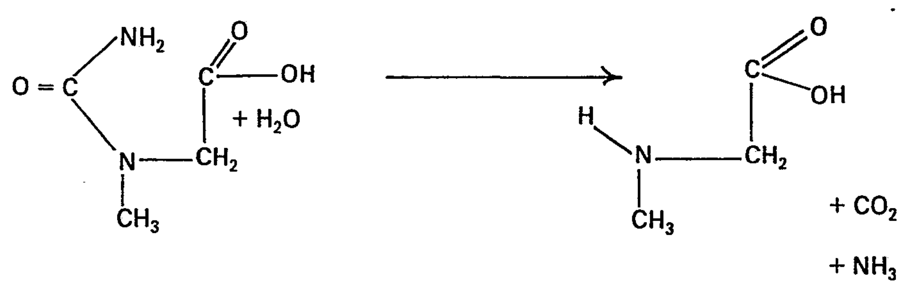

erhältlich aus Mikroorganismen wie Arthrobacter DSM 2563, DSM 2564, Micrococcus spec. DSM 2565 oder Moraxelle DSM 2562, die auf N-Methylhydantoin gezüchtet werden.

2. Verfahren zur Gewinnung von N-Carbamoylsarcosin-Amidohydrolase gemäss Anspruch 1, dadurch gekennzeichnet, dass man einen auf N-Methylhydantoin gezüchteten Mikroorganismus mit einem die Aufarbeitung lohnenden Gehalt an N-Carbamoylsarcosin-Amidohydrolase aufschliesst, dem vom Unlöslichen befreiten klaren Überstand 0,3 bis 0,5 Gew.% Polyäthylenimin zusetzt, den gebildeten Niederschlag entfernt und aus dem Überstand durch Verdünnen mit $H_2O$ die N-Carbamoylsarcosin-Amidohydrolase ausfällt und isoliert.

3. Verfahren nach Anspruch 2, dadurch gekennzeichnet, dass man einen Mikroorganismus der Gattung Arthrobacter, Micrococcus oder Moraxella verwendet.

4. Verfahren nach Anspruch 3, dadurch gekennzeichnet, dass man Arthrobacter DSM 2563, DSM 2564, Micrococcus spec. DSM 2565 oder Moraxella DSM 2562 verwendet.

5. Verfahren zur Bestimmung von N-Carbamoylsarcosin, dadurch gekennzeichnet, dass man eine die zu bestimmende Substanz enthaltende Probelösung mit N-Carbamoylsarcosin-Amidohydrolase gemäss Anspruch 1 in Sarcosin überführt und letzteres bestimmt.

6. Verfahren nach Anspruch 5, dadurch gekennzeichnet, dass die Sarcosinbestimmung mit Sarcosinoxidase und Messung von gebildetem $H_2O_2$ oder verbrauchtem $O_2$ durchgeführt wird.

7. Verfahren nach Anspruch 5 oder 6, dadurch gekennzeicnet, dass die Umsetzung in gepufferter Lösung bei einem pH-Wert zwischen 6 und 9 durchgeführt wird.

8. Reagenz zur Bestimmung von N-Carbamoylsarcosin, dadurch gekennzeichnet, dass es N-Carbamoylsarcosin-Amidohydrolase gemäss Anspruch 1, Sarcosinoxidase, ein System zum Nachweis von $H_2O_2$ und Puffer pH 6 bis 9 sowie gegebenenfalls ein oberflächenaktives Mittel enthält.

**Revendications**

1. N-carbamoylsarcosine-amidohydrolase, qui forme spécifiquement de la sarcosine à partir de la N-carbamoylsarcosine conformément à la formule suivante:

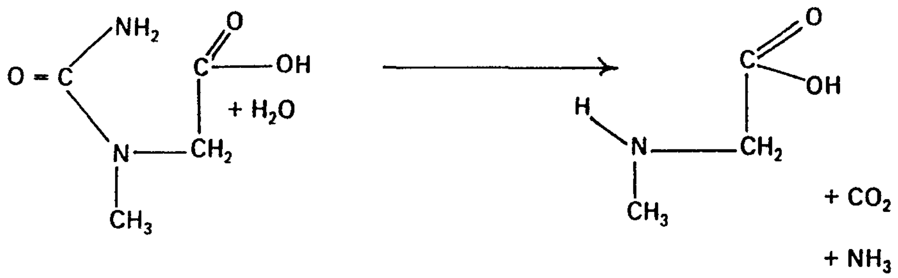

pouvant être obtenue à partir de microorganismes tels qu'Arthrobacter DSM 2563, DSM 2564, Micrococcus spec. DSM 2565 ou Moraxella DSM 2562, qui sont cultivés sur N-méthyl-hydantoïne.

2. Procédé d'obtention de la N-carbamoylsarcosine-amidohydrolase suivant la revendication 1, caractérisé en ce qu'on désagrège un microorganisme cultivé sur N-méthyl-hydantoïne ayant une teneur en N-carbamoylsarcosine-amidohydrolase justifiant le traitement, en ce qu'on ajoute au liquide surnageant limpide débarrassé des matières insolubles 0,3 à 0,5% en poids de polyéthylènimine, en ce qu'on élimine le précipité formé et en ce qu'on précipite la N-carbamoylsarcosine-amidohydrolase à partir du liquide surnageant par dilutoin avec $H_2O$ et on l'isole.

3. Procédé suivant la revendication 2, caractérisé en ce qu'on utilise un microorganisme du genre Arthrobacter, Micrococcus ou Moraxella.

4. Procédé suivant la revendication 3, caractérisé en ce qu'on utilise Arthrobacter DSM 2563, DSM 2564, Micrococcus spec. DSM 2565 ou Moraxella DSM 2562.

5. Procédé de dosage de la N-carbamoylsarcosine, caractérisé en ce qu'on transforme une solution d'échantillon contenant la substance à doser avec la N-carbamoylsarcosine-amidohydrolase suivant la revendication 1 en sarcosine et en ce qu'on dose cette dernière.

6. Procédé suivant la revendication 5, caractérisé en ce que le dosage de la sarcosine est effectué avec de la sarcosine-oxydase et avec mesure de l'$H_2O_2$ formée ou de l'$O_2$ consommé.

7. Procédé suivant la revendication 5 ou 6, caractérisé en ce que la réaction dans la solution tamponnée est effectuée à une valeur de pH entre 6 et 9.

8. Réactif pour le dosage de la N-carbamoylsarcosine, caractérisé en ce qu'il contient de la N-carbamoylsarcosine-amidohydrolase suivant la revendication 1, de la sarcosine-oxydase, un système pour la détection de $H_2O_2$ et un tampon à pH 6 à 9, ainsi que le cas échéant un agent tensioactif.

## Claims

1. N-Carbamoylsarcosine amidohydrolase, which specifically forms sarcosine from N-carbamoylsarcosine according to the following equation:

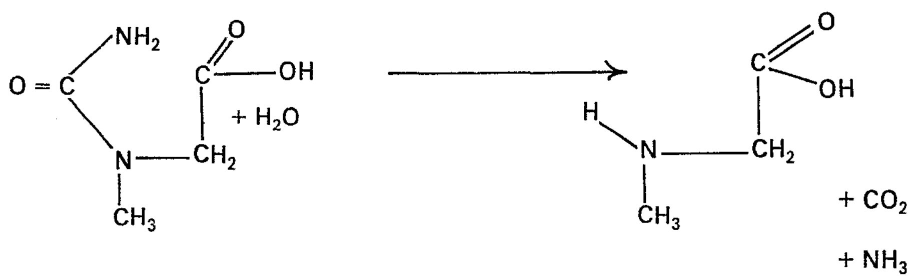

obtainable from micro-organisms, such as Arthrobacter DSM 2563, DSM 2564, Micrococcus spec. DSM 2565 or Moraxella DSM 2562, which are cultured on N-N-methyl-hydantoin.

2. Process for obtaining N-carbamoylsarcosine amidohydrolase according to claim 1, characterised in that one digests a micro-organism cultured on N-methyl-hydantoin with a content of N-carbamoylsarcosine amidohydrolase making working up worthwhile, adds to the clear filtrate, freed from insolubles, 0.3 to 0.5 wt.% polyethyleneimine, removes the precipitate formed and precipitates and isolates the carbamoylsarcosine amidohydrolase from the supernatant by dilution with $H_2O$.

3. Process according to claim 2, characterised in that one uses a micro-organism of the genus Arthrobacter, Micrococcus or Moraxella.

4. Process according to claim 3, characterised in that one uses Arthrobacter DSM 2563, DSM 2564, Micrococcus spec. DSM 2565 or Moraxella DSM 2562.

5. Process for the determination of N-carbamoylsarcosine, characterised in that one converts the substance to be determined in a sample solution with N-carbamoylsarcosine amidohydrolase according to claim 1 into sarcosine and determines the latter.

6. Process according to claim 5, characterised in that the sarcosine determination is carried out with sarcosine oxidase and measurement of the $H_2O_2$ formed or of consumed $O_2$.

7. Process according to claim 5 or 6, character-

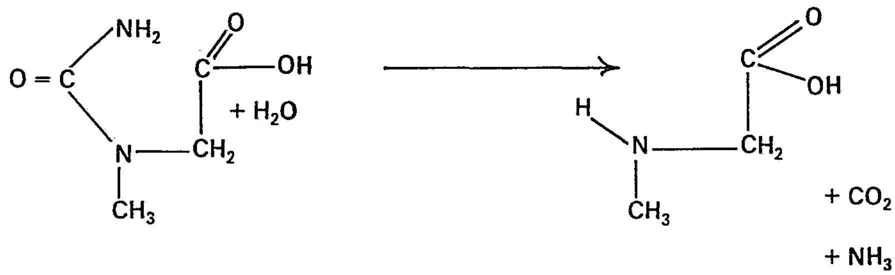

ised in that the reaction is carried out in buffered solution at a ph value between 6 and 9.

8. Reagent for the determination of N-carbamoylsarcosine, characterised in that it contains N-carbamoylsarcosine amidohydrolase according to claim 1, sarcosine oxidase, a system for the detection of $H_2O_2$ and buffer pH 6 to 9, as well as possibly a surface-active agent.